# EUROPEAN PATENT SPECIFICATION

(11) **EP 2 326 253 B1**
(45) Date of publication and mention of the grant of the patent: **23.11.2016**
(21) Application number: 09808650.7
(22) Date of filing: 17.08.2009
(51) Int. Cl.: A61B 10/02, A61B 18/00, A61M 1/00, A61B 6/00, A61B 5/055

(54) **INTEGRATED SURGICAL SAMPLING PROBE**
INTEGRIERTE CHIRURGISCHE PROBENENTNAHMESONDE
SONDE D ÉCHANTILLONNAGE CHIRURGICALE INTÉGRÉE

(30) Priority: 18.08.2008 US 136190 P
(43) Date of publication of application: 01.06.2011
(73) Proprietor: The Brigham and Women's Hospital, Inc., Boston, MA 02115 (US)
(72) Inventor: JOLESZ, Ferenc, Brookline MA 02445 (US); GOLBY, Alexandra, Boston MA 02120 (US); AGAR, Nathalie, Newton MA 02459 (US)
(74) Representative: Gill Jennings & Every LLP
(86) International application number: PCT/US2009/053967
(87) International publication number: WO 2010/021951

(56) References cited:
- WO-A1-00/25692
- JP-A- 10 137 249
- JP-A- 2001 104 315
- US-A- 4 750 902
- US-A- 4 827 911
- US-A1- 2005 049 486
- US-A1- 2007 239 153
- US-A1- 2008 058 672
- US-A1- 2008 146 965

## Description

### Field of the Invention

The present invention is directed to medical devices that can be used to obtain tissue samples for diagnostic evaluation and which are integrated with a stereotactic coordinate system so that the exact location of biopsy sites can be determined.

### Background of the Invention

Stereotactic surgical procedures were developed in the early 1900's and were first applied clinically in the 1940's (Kelly, P., Nearrosarrgery 46:16 (2000)). Initially these procedures were used in neurosurgery and involved affixing an external apparatus to a patient's skull to establish a coordinate system for locating, in a reproducible manner, the exact position of a lesion within the intracranial area. Today, stereotactic procedures have been applied to other tissues and are typically used in conjunction with diagnostic imaging procedures such as CT scans and MRIs to map internal tissues, prior to, or during, surgery (see, *e.g.,* Poza, et al., Appl. Neurophysiol., 48:482-487 (1985); Dorwald, et al. Br. J. Neurosurg. 16:110-118 (2002); Krieger, et al., J. Surg. Oncol. 14:13-25 (1998)).

The development of stereotactic methods and imaging techniques has been accompanied by the development of surgical instruments that allow physicians to perform procedures at sites that were formerly inaccessible. Among the most successful of the instruments that have been developed for neurosurgery are probes designed to ultrasonically ablate tissue. For example, the Cavitron Ultrasonic Surgical Aspirator® (Integra Radionics) uses pulses of ultrasonic energy delivered to a needle-like tip to fragment tissue, which is concurrently irrigated and removed by aspiration. Although probes of this type were initially designed primarily for the surgical resection of tumors, it was subsequently found that the tissue fragments generated by the devices maintain sufficient integrity to be used diagnostically (Richmond, et al., Neurosurg. 13:415-419 (1983); Malhotra, et al., Acta Neurochir. 81:132-134 (1986); Blackie, et al., J. Clin. Pathol. 37:1101-1104 (2008)).

In addition to probes that ablate tissue ultrasonically, probes such as the Nico Myriad™ probe (NICO Corporation) have been designed to perform surgical ablations by mechanically cutting or shaving tissue. One attractive aspect of these "mechanical sampling" probes is that tissue is obtained without the generation of heat.

Despite the advances noted above, the diagnostic use of ultrasonic and mechanical probes has gone largely undeveloped and potential advantages over traditional methods of tissue sampling have often gone unrecognized.

### Summary of the Invention

The present invention is based upon the concept of integrating a tissue resection device with a stereotactic navigation system and then using the device to collect tissue fragments for diagnostic assays. This allows tissue sampling locations to be precisely determined. Preliminary results and published articles reporting on the histopathological evaluation of tissue fragments indicate that ultrasonically generated fragments preserve the features required for standard histopathological diagnosis. It is expected that mechanically generated fragments would also preserve these features.

The invention is defined in claim 1. Preferred embodiments are defined in dependent claims 2-10. A medical device according to the preamble of claim 1 is known from US-A-4 750 902. In its first aspect, the invention is directed to a medical device (also referred to herein as a "probe") that can be used in collecting tissue samples from biopsy sites in a patient. Examples of devices that may be adapted for this purpose can be found in US 4,827,911; 4,861,332; 4,660,573; 4,320,761; 4,561,438; 4,169,984; 4,223,676; 4,425,115; and 7,137,963. The device includes a hand held support, typically made of plastic, metal or rubber with a shape and size that allows it to be easily held and maneuvered in an operator's hand. Typically, these supports will have a rectangular or cylindrical shape and be about 4 to 8 inches in length, although other shapes and sizes are possible. Extending from, and attached to, one end of the hand held support is an elongated metal rod with a proximal end (the end attached to the support) and a distal end (the end furthest from the support). The rod will typically be about 3 to 10 inches long and terminate at its distal end in a tip that either itself vibrates in response to ultrasonic energy or which has a separate component attached to it that vibrates in response to ultrasonic energy.

The medical device also includes means for supplying ultrasonic energy to the tip or to the separate vibrational component, preferably at a frequency of 15-100 kHz and, more preferably, at 20-60 kHz. Systems for generating ultrasonic vibrations have been known in the art for many years and have been used in many types of devices (see *e.g.,* US Patent Nos. 2,594,841; 3,975,650; 4,736,130; 4,868,445; 5,076,854; 5,276,376; and 5,496,411.

In addition, the device includes means for supplying irrigating fluid to the distal end of the tip and for aspirating tissue fragments created at the tip as the result of ultrasonic vibrations (see *e.g.,* US 3,693,613; 4,063,557 and 4,827,911. A preferred method for supplying irrigating fluid is by pumping it from a reservoir through a tubular channel running through the rod and terminating in an opening at the tip. The exact diameter of the channel is not critical to the invention but will typically be between 3.175 mm and 12.7 mm (1/8 and 1/2 of an inch). The reservoir may contain any pharmaceutically acceptable fluid such as water, saline, Ringer's solution etc. and may be maintained at room temperature or chilled, *e.g.,* to 0-15°C. If desired, the fluid may also include antibiotics to help prevent infection or other drugs.

With respect to aspiration, it is preferred that the metal rod of the device have a hollow core that provides a fluid passageway for tissue fragments. This passageway is open at the tip and extends through or past the hand held support of the device. Sufficient suction is provided, *e.g.,* by means of a medical suction pump, to aspirate material through the opening at the tip in the direction of the hand held support. As with the channel for providing irrigation fluid, the diameter of the passageway for aspiration is not critical but will, in general, be between 3.175 mm and 12.7 mm (1/8 and 1/2 inch).

The passageway is preferably connected at its proximal end to a tissue collection container where aspirated fragments are delivered and which, in some embodiments, contains a fluid such as water, saline or Ringer's solution. This fluid may, optionally be chilled, *e.g.,* to 0-10°C, and/or may include chemicals for fixing tissue samples. Examples of irrigation and aspiration systems may be found in US 6,723,110; 6,565,535; 4,504,264; 4,531,934; and4,637,814 as well as in references cited above.

A primary improvement with respect to the present medical device is that it is integrated with a system for stereotactically determining the position of the distal end of the rod (*i.e.,* the location where aspirated tissue samples are collected) relative to the tissue being examined (*e.g*., brain tissue). Any of the stereotactic positioning systems that are known in the art may be adapted for this purpose. Examples of such systems and related materials are described in US 4,618,978; 5,171,296; 5,207,688; 5,311,868; 5,383,454; 5,423,832; 5,531,229; 5,628,315; 5,665,095; 5,690,108; 5,706,811; 5,728,106; 5,947,981; and 6,355,049.

The stereotactic system preferably includes a computer that stores information regarding the spatial relationship between the probe (particularly the tip of the probe) and the tissue of the patient being examined. The probe includes means for communicating information to the computer regarding its position. This may be accomplished using, *inter alia*: a) ultrasound detectors (see *e.g.,* US 7,251,352; 7,142,905; b) electromagnetic emitters located on the device (preferably on the hand held support of the device) that transmit signals to a separate electromagnetic receiver (see US 7,357,573; 7,313,430; c) sound emitters located on the device (preferably on the hand held support) that transmit signals to microphones (see US 6,374,135; or d) by "optical tracking using infrared energy detectors (see US 7,302,288). In each instance, signals are communicated to the computer for analysis. The most preferred method for communicating information concerning the position of the device is with electromagnetic emitters.

In a preferred embodiment, the hand held support includes an actuator switch which, when activated, permits the transmission of ultrasonic energy to the tip of the rod or to a separate component which vibrates in response to ultrasonic energy. When the switch is not activated ultrasonic energy is not transmitted. Activation of the actuator switch is, preferably, accompanied by the transmission of a signal to the computer to aid in determining the position of the tip of the rod at the time of actuation. The actuator switch may be formed of a foot pedal which, when activated, transmits ultrasonic energy to the tip of the rod.

### Brief Description of the Drawings

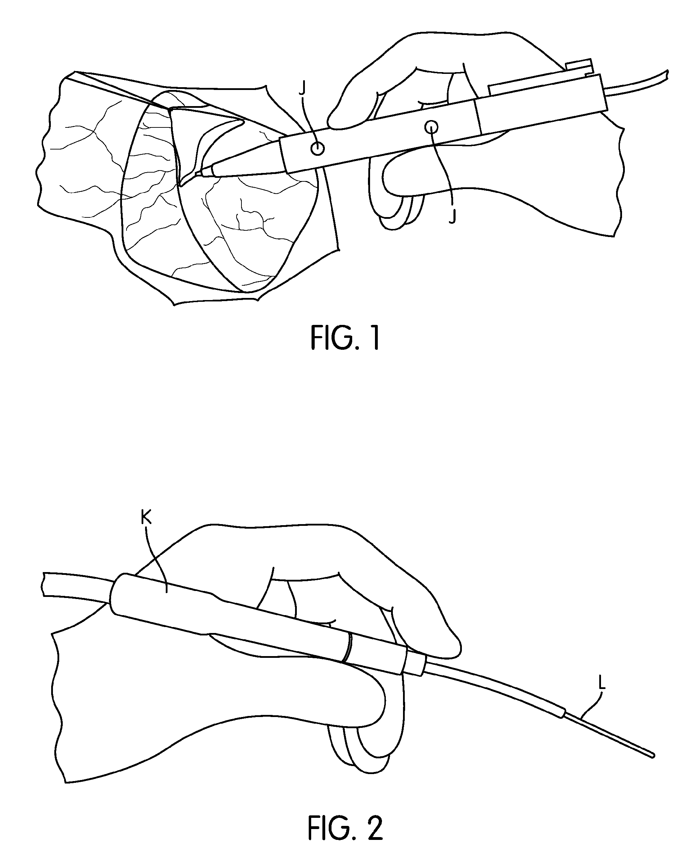

Figure 1: Figure 1 illustrates a type of probe that may be adapted for use in the present invention. The probe disrupts cellular tissue through longitudinal vibration of a hollow tip at ultrasonic frequencies (24 or 35 kHz). Combining the disruption process with irrigation coming from the annular space surrounding the probe assists in removal of the tissue by aspiration through the center of the handpiece into a waste container. For the purposes of the present invention, the waste container is replaced with a collection container and/or analytical device. The figure shows two electromagnetic sensors (J) that have been located on the hand held support of the device for signaling its position.
Figure 2: Figure 2 is a drawing of a device that has a hand held support (K) and an elongated metal rod (L).

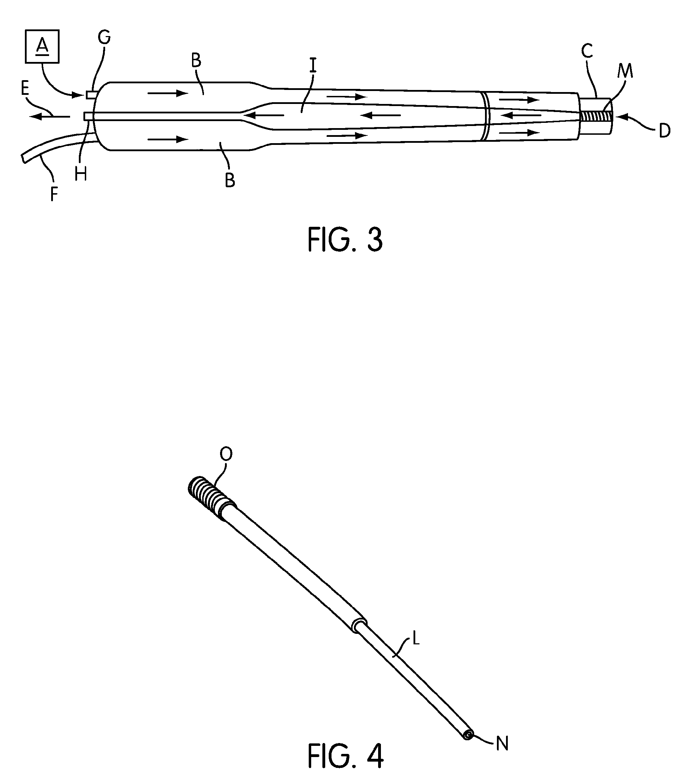

Figure 3: Figure 3 is an illustration of a hand held support for a device showing a channel for the flow of irrigation fluid (B); and an opening (D) leading into a passageway for aspirated tissue fragments (I). A reservoir of irrigation fluid is represented as (A) and feeds into the channel B through port (G). The aspirated tissue exits the passageway carrying aspirated tissue (I) in a stream (E) passing through port (H) and may be collected in any suitable specimen container. At the distal end of the hand held support is a coupling region (C) where the elongated rod may be attached. In the embodiment shown, attachment is accomplished using threaded bore (M). The figure also shows (F) which represents a cord for transmitting energy that causes the tip of the device to vibrate.
Figure 4: Figure 4 shows the terminal part of a device that includes an elongated metal rod L terminating in an opening (N), through which tissue samples may be aspirated. In the embodiment shown, the rod is designed to attach to the hand held support by means of a threaded regions at its base (O) that can be screwed into a matching region in the hand held support (M in Figure 3).

### Detailed Description of the Invention

The present invention is directed to systems for obtaining tissue samples that record the exact location where each sample is taken. Existing devices that have previously been used primarily for tissue ablation may be adapted for the retrieval of tissue samples and modified so that they integrate with existing systems for the stereotactic positioning of medical devices.

Devices that utilize ultrasonic vibrations to fragment tissues and that recover the fragments by aspiration are one type of device that is particularly well suited to the invention because fragments produced in this manner have been shown to retain histological characteristics that can be used diagnostically. An example of such a device is the Cavitron Ultrasonic Surgical Aspirator (CUSA), made by Integra Radionics and similar to the device illustrated in Figure 2. The hand held base unit of the device is shown as (K) with a curved and elongated metal rod (L) extending from it. Devices of this type can be modified by incorporating a component into the hand held support that will provide a signal that can be used in analyzing its exact position. For example, electromagnetic emitters may be included in the support to provide a signal to a separate receiver, which, in turn, communicates this information to a computer for analysis. A drawing of a device with electromagnetic emitters (J) is shown in Figure 1. Other signaling systems that may be used include those that detect ultrasonic signals, sound signals and infrared signals.

Devices should also include means for irrigating and aspirating tissues after fragmentation. This is illustrated in Figure 3 which shows the hand held base unit of a device. In this figure, (A) represents a reservoir containing irrigation fluid and is connected to a port (G) leading into a channel (B). The channel runs to the distal end of the device where fluid exits and flows or sprays onto tissue. The end of the hand held base unit (C) attaches to the elongated rod (shown in Figure 4) which vibrates at its tip in response to ultrasonic energy provided by an ultrasonic energy generator and transmitted via cord (F). The end of the base unit has an opening (N) that leads into a passageway (I in Figure 3) extending from the tip of the rod to a port (H) at the opposite end of the support unit. Aspirated tissue exits this port in a stream (E) and may be delivered to a collection container. This container may contain fluids such as water or saline to preserve the tissue fragments and may optionally be chilled or contain fluid for fixing tissue. Samples recovered from the collection container may then be examined for histological features characteristic of disease or used in other diagnostic tests.

In order for the devices described above to provide information on the location of sampling sites, they can be integrated with existing systems for the stereotactic analysis of spatial arrangements. Many stereotactic systems have been developed (primarily to aid in neurosurgery) and can readily be adapted for the present invention. The first step in using these systems is to establish a three dimensional stereotactic coordinate system for reproducibly identifying positions in the tissue of the patient. This is usually accomplished using an apparatus or electrodes that are placed in fixed positions on the patient as a frame of reference. Diagnostic imaging procedures *(e.g.,* CT scans or MRI scans) may then be performed to provide information concerning the internal tissues of the patient and the spatial relationship of the tissues to the established coordinate system. For example, imaging procedures may be used to provide information on the exact location of a tumor. After imaging, an important step is the registration step which takes place in the OR.

### Examples

We collected brain tumor specimens using both, surgical forceps and CUSA and then performed histopathological and mass spectrometry analyses. The histopathology showed preservation of histology features required for diagnosis, and the direct mass spectrometry analysis of the tissue specimens using a DESI-LTQ instrument revealed molecular signatures indicative of neoplasia, as compared to specimens biopsied using surgical forceps. This new integrated surgical-sampling probe will enable the differentiation of tumor from non-tumor tissue based on measurements or imaging of the samples.

## Claims

1. A medical device comprising:
a) a hand held support (K);
b) an elongated metal rod (L) extending from and attached to said hand-held support (K), wherein said rod terminates at its distal end in a tip that either itself vibrates in response to ultrasonic energy or comprises a separate component which vibrates in response to ultrasonic energy;
c) means for supplying ultrasonic energy to said tip or said separate component (F);
d) means for supplying irrigating fluid to said tip;
e) means for aspirating tissue fragments created at said tip;
f) means for collecting tissue samples, **characterised in**
g) means for stereotactically determining position of said tip.

2. The medical device of claim 1, wherein said means for stereotactically determining the position of the tip, comprises one or more electromagnetic emitters (J) located on said device that transmit signals to a separate electromagnetic receiver or infrared energy detectors for optical tracking.

3. The medical device of claim 1 wherein said rod has a hollow core (N) that provides a fluid passageway (I) running along the length of said rod to said tip.

4. The medical device of claim 3, wherein said aspirating means creates suction in said
hollow core of said rod to aspirate material through said tip in the direction of said hand held support.

5. The medical device of claim 4, wherein said fluid passageway (I) terminates at its proximal end in a tissue collection container (E).

6. The medical device of claim 5, wherein said tissue collection container is chilled or comprises a fluid for fixing tissue samples.

7. The medical device of claim 1, wherein said means for supplying ultrasonic energy operates at a frequency of 15-100 kHz.

8. The medical device of claim 1, wherein said means for supplying irrigating fluid comprises a tubular channel (B) running through said rod, wherein said channel is connected at its proximal end to a reservoir of irrigating fluid (A) and wherein said channel terminates at the tip of said rod (L) in an opening through which said irrigating fluid can flow.

9. The medical device of claim 1, wherein said hand held support comprises an actuator switch which, when activated, permits the transmission of ultrasonic energy to the tip of said rod (L) or to said separate component which vibrates in response to ultrasonic energy and which does not permit said transmission when not activated.

10. The medical device of claim 9, wherein, said means for stereotactically determining the position of the distal end of said rod, comprises electromagnetic emitters (J) located on said device that transmit signals to a separate electromagnetic receiver, wherein said electromagnetic receiver communicates signal data to a computer programmed to analyze position or infrared energy detectors for optical tracking.

## Patentansprüche

1. Ein Medizinprodukt, das Folgendes beinhaltet:
a) eine Handhaltevorrichtung (K);
b) eine längliche Metallstange (L), die sich aus der Handhaltevorrichtung (K) erstreckt und an ihr befestigt ist, wobei die Stange an ihrem distalen Ende in einer Spitze endet, die entweder selbst als Reaktion auf Ultraschallenergie vibriert oder eine separate Komponente beinhaltet, die als Reaktion auf Ultraschallenergie vibriert;
c) Mittel zum Zuführen von Ultraschallenergie zu der Spitze oder zu der separaten Komponente (F);
d) Mittel zum Zuführen von Spülfluid zu der Spitze;
e) Mittel zum Aspirieren von an der Spitze geschaffenen Gewebefragmenten;
f) Mittel zum Sammeln von Gewebeproben, **gekennzeichnet durch**
g) Mittel zum stereotaktischen Ermitteln der Position der Spitze.

2. Das Medizinprodukt gemäß Anspruch 1, wobei das Mittel zum stereotaktischen Ermitteln der Position der Spitze einen oder mehrere auf dem Medizinprodukt befindliche elektromagnetische Sender (J) beinhaltet, die Signale zu einem separaten elektromagnetischen Empfänger oder Infrarotenergiedetektoren zur optischen Nachverfolgung übertragen.

3. Das Medizinprodukt gemäß Anspruch 1, wobei die Stange einen hohlen Kern (N) aufweist, der einen Fluiddurchgang (I) bereitstellt, der an der Länge der Stange entlang bis zu der Spitze verläuft.

4. Das Medizinprodukt gemäß Anspruch 3, wobei das Aspirationsmittel Saugwirkung in dem hohlen Kern der Stange schafft, um Material durch die Spitze in die Richtung der Handhaltevorrichtung zu aspirieren.

5. Das Medizinprodukt gemäß Anspruch 4, wobei der Fluiddurchgang (I) an seinem proximalen Ende in einem Gewebesammelbehälter (E) endet.

6. Das Medizinprodukt gemäß Anspruch 5, wobei der Gewebesammelbehälter gekühlt ist oder ein Fluid zum Fixieren von Gewebeproben beinhaltet.

7. Das Medizinprodukt gemäß Anspruch 1, wobei das Mittel zum Zuführen von Ultraschallenergie bei einer Frequenz von 15-100 kHz betrieben wird.

8. Das Medizinprodukt gemäß Anspruch 1, wobei das Mittel zum Zuführen von Spülfluid einen rohrförmigen Kanal (B) beinhaltet, der durch die Stange verläuft, wobei der Kanal an seinem proximalen Ende mit einem Spülfluidreservoir (A) verbunden ist und wobei der Kanal an der Spitze der Stange (L) in einer Öffnung endet, durch die das Spülfluid fließen kann.

9. Das Medizinprodukt gemäß Anspruch 1, wobei die Handhaltevorrichtung einen Betätigungsschalter beinhaltet, der, wenn er aktiviert ist, die Übertragung von Ultraschallenergie zu der Spitze der Stange (L) oder zu der separaten Komponente, die als Reaktion auf Ultraschallenergie vibriert, erlaubt und der, wenn er nicht aktiviert ist, die Übertragung nicht erlaubt.

10. Das Medizinprodukt gemäß Anspruch 9, wobei das Mittel zum stereotaktischen Ermitteln der Position des distalen Endes der Stange elektromagnetische Sender (J) beinhaltet, die Signale zu einem separaten elektromagnetischen Empfänger übertragen, wobei der elektromagnetische Empfänger Signaldaten an einen Computer übermittelt, der zum Analysieren der Position oder Infrarotenergiedetektoren zur optischen Nachverfolgung programmiert ist.

## Revendications

1. Dispositif médical comportant:
a) un support tenu à la main (K)';
b) une tige métallique allongée (L) s'étendant à partir du support tenu à la main (K) et fixé à ce support, **caractérisé en ce que** la tige se termine à l'extrémité distale par un embout qui vibre en réponse à l'énergie ultrasonique ou bien qui comprend un composant distinct qui vibre en réponse à l'énergie ultrasonique;
c) un moyen de fournir de l'énergie ultrasonique à cet embout ou au composant distinct (F);
d) un moyen de fournir le fluide d'irrigation à cet embout;
e) un moyen d'aspirer les fragments de tissu créés au niveau de l'embout;
f) un moyen de rassembler les échantillons de tissu, **caractérisé par**
g) un moyen de déterminer de manière stéréotaxique la position de cet embout.

2. Dispositif médical selon la revendication 1, **caractérisé en ce que** le moyen servant à déterminer de manière stéréotaxique la position de l'embout comprend un ou plusieurs émetteurs électromagnétiques (J) situés sur le dispositif, et qui transmettent des signaux vers un récepteur électromagnétique distinct, ou bien vers des détecteurs d'énergie à infrarouge pour les besoins de poursuite optique.

3. Dispositif médical selon la revendication 1, **caractérisé en ce que** la tige comprend un noyau creux (N) qui sert de passage pour le fluide (I) le long de la tige et vers l'embout.

4. Dispositif médical selon la revendication 3, **caractérisé en ce que** le moyen d'aspiration crée une aspiration dans le noyau creux de la tige qui fera aspirer le matériau å travers l'embout en direction du support tenu à la main.

5. Dispositif médical selon la revendication 4, **caractérisé en ce que** le passage de fluide (I) se termine à son extrémité proche par un récipient de rassemblement de tissus (E).

6. Dispositif médical selon la revendication 5, **caractérisé en ce que** le récipient de rassemblement de tissus est refroidi ou bien **en ce qu'**il contient un fluide servant à fixer les échantillons de tissu.

7. Dispositif médical selon la revendication 1, **caractérisé en ce que** le moyen de fournir l'énergie ultrasonique fonctionne à la fréquence de 15-100 kHz.

8. Dispositif médical selon la revendication 1, **caractérisé en ce que** le moyen de fournir le fluide d'irrigation comprend une voie tubulaire (B) disposée à travers la tige, **caractérisé en ce que** cette voie est raccordée à son extrémité proche à un réservoir de fluide d'irrigation (A), et **caractérisé en ce que** la voie se termine au niveau de l'embout de la tige (L) par une ouverture à travers laquelle peut s'écouler le fluide d'irrigation.

9. Dispositif médical selon la revendication 1, **caractérisé en ce que** le support tenu à la main comprend un actionneur qui, lorsqu'il est mis en oeuvre, permet de transmettre l'énergie ultrasonique à l'embout de la tige (L) ou au composant distinct qui vibre en réponse à l'énergie ultrasonique, et qui ne permet pas cette transmission lorsqu'il n'est pas mis en oeuvre.

10. Dispositif médical selon la revendication 9, **caractérisé en ce que** le moyen servant à déterminer de manière stéréotaxique la position de l'extrémité la plus éloignée de la tige comprend des émetteurs électromagnétiques (J) situés sur le dispositif qui transmettent des signaux vers un récepteur électromagnétique distinct, **caractérisé en ce que** l'émetteur électromagnétique transmet les signaux vers un ordinateur qui est programmé de manière à analyser la position de détecteurs d'énergie à infrarouge pour les besoins de poursuite optique.
